(19) Europäisches Patentamt
European Patent Office
Office européen des brevets

(11) Veröffentlichungsnummer: **0 235 725 B1**

(12) # EUROPÄISCHE PATENTSCHRIFT

(45) Veröffentlichungstag der Patentschrift: **21.11.91**

(21) Anmeldenummer: **87102571.4**

(22) Anmeldetag: **24.02.87**

(51) Int. Cl.⁵: **C07D 401/06**, C07D 417/06, C07D 403/06, C07D 277/32, C07D 413/06, A01N 43/50, A01N 43/78, A01N 43/60, A01N 43/40, A01N 43/56, A01N 43/76

(54) **Heterocyclische Verbindungen.**

(30) Priorität: **07.03.86 JP 48629/86**

(43) Veröffentlichungstag der Anmeldung:
**09.09.87 Patentblatt 87/37**

(45) Bekanntmachung des Hinweises auf die Patenterteilung:
**21.11.91 Patentblatt 91/47**

(84) Benannte Vertragsstaaten:
**AT BE CH DE ES FR GB GR IT LI NL**

(56) Entgegenhaltungen:
**FR-A- 2 542 740**
**GB-A- 2 126 220**

**PATENT ABSTRACTS OF JAPAN Band 9, Nr. 241 (C-306)(1964), 27. September 1985; & JP - A - 60 100 557**

**PATENT ABSTRACTS OF JAPAN Band 8, Nr. 122 (C-227)(1559), 8. Juni 1984; & JP - A - 59 33 283**

(73) Patentinhaber: **NIHON BAYER AGROCHEM K.K.**
**7-1, Nihonbashi Honcho 2-chome Chuo-ku**
**Tokyo 103(JP)**

(72) Erfinder: **Shiokawa, Kozo**
**210-6 Shukugawara Tama-ku**
**Kawasaki-shi Kanagawa-ken(JP)**
Erfinder: **Tsuboi, Shinichi**
**3-26-1, Hirayama**
**Hino-shi Tokyo(JP)**
Erfinder: **Kagabu, Shinzo**
**1768-Sagiyama**
**Gifu-chi Gifu-ken(JP)**
Erfinder: **Sasaki, Shoko**
**1-7-3, Higashi-Hirayama**
**Hino-shi Tokyo(JP)**
Erfinder: **Moriya, Koichi**
**39-15, Namiki-cho**
**Hachioji-shi Tokyo(JP)**
Erfinder: **Hattori, Yumi**
**598, Kobiki-cho**
**Hachioji-shi Tokyo(JP)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 235 725 B1

(74) Vertreter: **Schumacher, Günter, Dr. et al**
**c/o Bayer AG Konzernverwaltung RP Patent-abteilung**
**W-5090 Leverkusen 1 Bayerwerk(DE)**

## Beschreibung

Die vorliegende Erfindung betrifft neue heterocyclische Verbindungen, Verfahren zu ihrer Herstellung und ihre Verwendung als Insektizide.

Es ist bereits bekannt, daß bestimmte cycloiminosubstituierte heterocyclische Verbindungen als Zwischenprodukte für fungizide, antidiabetische, viruzide, tranquilisierende oder diuretische aktive Substanzen (siehe die DE-OS 2 205 745) und auch als Antiulcus-Mittel (siehe die DE-OS 3 409 801) verwendbar sind.

Weiterhin ist aus den Publikationen GB-A 2 126 220; Japan Abstr., Vol. 9, 241, 1985 und Japan Abstr., Vol. 8, 122, 1984 die ebenfalls als Antiulcus-Mittel einsetzbare Verbindung der Formel

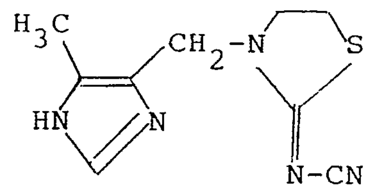

bekannt.

Es wurden nun neue heterocyclische Verbindungen der Formel (I)

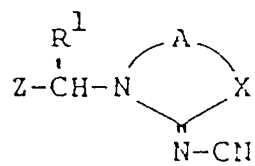

(I)

gefunden, in der

- $R^1$ ein Wasserstoff-Atom oder eine Methyl-Gruppe bezeichnet,
- A eine Ethylen-Gruppe, die durch Methyl substituiert sein kann, oder eine Trimethylen-Gruppe, die durch Methyl substituiert sein kann, bezeichnet,
- X ein Sauerstoff- oder Schwefel-Atom oder die Gruppe

$$-N-R^2$$

oder

$$-CH-R^3$$

bezeichnet, worin

- $R^2$ ein Wasserstoff-Atom, eine $C_1$-$C_4$-Alkyl-Gruppe, die durch einen aus Halogenen, $C_1$-$C_4$-Alkoxy-Gruppen, $C_1$-$C_4$-Alkylthio-Gruppen und Cyano ausgewählten Substituenten substituiert sein kann, eine $C_2$-$C_4$-Alkenyl-Gruppe, eine $C_2$-$C_4$-Alkinyl-Gruppe, eine Pyridylmethyl-Gruppe, die durch Halogen und/oder Methyl substituiert sein kann, eine Benzyl-Gruppe, die durch Halogen und/oder Methyl substituiert sein kann, eine Formyl-Gruppe, eine Alkylcarbonyl-Gruppe mit 1 bis 2-Kohlenstoff-Atomen in der Alkyl-Struktureinheit, die durch Halogen substituiert sein kann, eine Phenylcarbonyl-Gruppe, die durch Halogen und/oder Methyl substituiert sein kann, eine Alkoxy- oder Alkylthiocarbonyl-Gruppe mit 1 bis 4-Kohlenstoff-Atomen in der Alkyl-Struktureinheit, eine Phenoxycarbonyl-Gruppe, eine $C_1$-$C_4$-Alkylsulfonyl-Gruppe, die durch Halogen substituiert sein kann, oder eine Phenylsulfonyl-Gruppe, die durch Methyl substituiert sein kann, bezeichnet und
- $R^3$ ein Wasserstoff-Atom bezeichnet, und
- Z eine 5- oder 6-gliedrige heterocyclische Gruppe, die zwei oder drei aus Sauerstoff-, Schwefel- und Stickstoff-Atomen ausgewählte Hetero-Atome enthält, von denen wenigstens eines ein Stickstoff-Atom ist, oder eine 3-Pyridyl-Gruppe bezeichnet, wobei die heterocyclische Gruppe und

die 3-Pyridyl-Gruppe gegebenenfalls durch wenigstens einen aus Halogen-Atomen, Alkyl-Gruppen mit 1 bis 4 Kohlenstoff-Atomen, Alkoxy-Gruppen mit 1 bis 4 Kohlenstoff-Atomen, Alkylthio-Gruppen mit 1 bis 4 Kohlenstoff-Atomen, Halogenoalkyl-Gruppen mit 1 bis 4 Kohlenstoff-Atomen, Halogenoalkoxy-Gruppen mit 1 bis 4 Kohlenstoff-Atomen, Alkylsulfonyl-Gruppen mit 1 bis 4 Kohlenstoff-Atomen, der Cyano-Gruppe und der Nitro-Gruppe ausgewählten Substituenten substituiert sind; mit Ausnahme der Verbindung der Formel

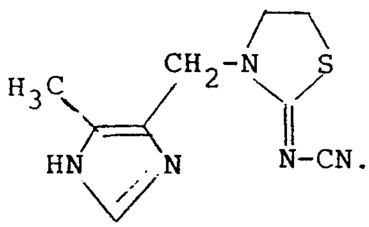

Die Verbindungen der Formel (I) werden mit Hilfe eines Verfahrens erhalten, bei dem

a) Verbindungen der Formel (II)

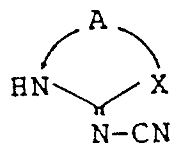

(II)

,

in der A und X die angegebenen Bedeutungen haben, mit Verbindungen der Formel (III)

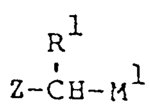

(III)

,

in der $R^1$ und Z die oben angegebenen Bedeutungen haben und $M^1$ für ein Halogen-Atom oder die Gruppe $-OSO_2-M^2$ steht, in der $M^2$ eine niedere Alkyl-Gruppe oder eine Aryl-Gruppe bezeichnet, in Gegenwart eines inerten Lösungsmittels und gegebenenfalls in Gegenwart einer Base umgesetzt werden oder,

b) in dem Fall, in dem X in der Formel (I) ein Sauerstoff- oder Schwefel-Atom oder die Gruppe

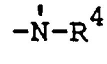

bezeichnet, in der $R^4$ für ein Wasserstoff-Atom, eine Alkyl-Gruppe, die substituiert sein kann, eine Alkenyl-Gruppe oder eine Alkinyl-Gruppe steht, wobei in diesem Fall in der nachstehenden Formel (IV) X durch das Symbol $X^1$ ersetzt ist,

Verbindungen der Formel (IV)

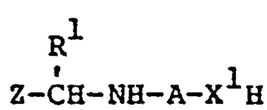

(IV)

,

in der $R^1$, A, Z und $X^1$ die angegebenen Bedeutungen haben, mit Verbindungen der Formel (V)

$$(R'-S)_2C=N-CN \qquad (V)$$

,

in der R' eine niedere Alkyl-Gruppe oder eine Benzyl-Gruppe bezeichnet oder zwei R'-Gruppen zusammen eine niedere Alkylen-Gruppe mit wenigstens zwei Kohlenstoff-Atomen bezeichnen und zusammen mit den benachbarten Schwefel-Atomen einen Ring bilden können,
in Gegenwart inerter Lösungsmittel umgesetzt werden oder,
c) in dem Fall, in dem X in der Formel (I) die Gruppe

$$-\overset{|}{N}-R^2$$

bezeichnet, in der $R^2$ für eine Acyl-Gruppe steht, die substituiert sein kann, wobei in diesem Fall in der nachstehenden Formel (VI) $R^2$ durch das Symbol $R^5$ ersetzt ist,
Verbindungen der Formel (Ib)

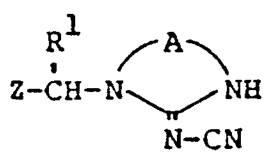

(Ib)

,

in der $R^1$, A und Z die oben angegebenen Bedeutungen haben, mit Verbindungen der Formel (VI)

$$R^5-Hal \qquad (VI)$$

,

in der $R^5$ die oben angegebene Bedeutung hat und Hal ein Halogen-Atom bezeichnet,
in Gegenwart inerter Lösungsmittel und einer Base umgesetzt werden.

Die neuen heterocyclischen Verbindungen der Formel (I) zeigen starke insektizide Eigenschaften.

Überraschenderweise zeigen die neuen heterocyclischen Verbindungen gemäß der Erfindung eine wesentlich größere insektizide Wirkung als diejenigen, die aus dem oben angeführten Stand der Technik bekannt sind, und insbesondere entfalten die Verbindungen eine außerordentlich stark überlegene Wirksamkeit als Insktizide gegen stechende und saugende Insekten, wie sie typisch durch Insekten der Gattung Hemiptera repräsentiert werden, etwa durch Blattläuse, Laternenträger, Wanzen und Heuschrecken, die infolge einer Langzeit-Anwendung Resistenz gegen Organophosphat- und Carbamat-Insektizide erworben haben.

Bevorzugte Verbindungen der Formel (I) sind diejenigen, in denen

- $R^1$ ein Wasserstoff-Atom bezeichnet,
- A eine Ethylen- oder Trimethylen-Gruppe bezeichnet,
- X ein Schwefel-Atom oder die Gruppe -NH bezeichnet und
- Z eine 5- oder 6-gliedrige heterocyclische Gruppe, die zwei aus Sauerstoff-, Schwefel- und Stickstoff-Atomen ausgewählte Hetero-Atome enthält, von denen wenigstens eines ein Stickstoff-Atom ist, oder eine 3-Pyridyl-Gruppe bezeichnet, wobei die heterocyclische Gruppe und die 3-Pyridyl-Gruppe gegebenenfalls durch wenigstens einen aus dem Fluor-Atom, dem Chlor-Atom, dem Brom-Atom, der Methyl-Gruppe, der Methoxy-Gruppe, der Methylthio-Gruppe, der Trifluoromethyl-Gruppe, der Trifluoromethoxy-Gruppe, der Methylsulfonyl-Gruppe, der Cyano-Gruppe und der Nitro-Gruppe ausgewählten Substituenten substituiert sind, mit Ausnahme der Verbindung der Formel

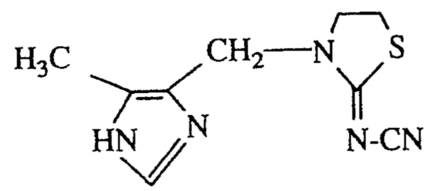

.

Die 3-Pyridyl-Gruppe in der Definition von Z ist mit 5-Pyridyl strukturell synonym.

Zu speziellen Beispielen der Verbindungen der Formel (I) gemäß der vorliegenden Erfindung zählen insbesondere
1-(2-Chloro-5-pyridylmethyl)-2-cyanoiminoimidazolidin,
1-(2-Fluoro-5-pyridylmethyl)-2-cyanoiminoimidazolidin,
1-(2-Chloro-5-pyridylmethyl)-2-cyanoiminotetrahydropyrimidin,
1-(2-Methyl-5-pyridylmethyl)-2-cyanoiminoimidazolidin,
1-(2-Chloro-5-thiazolylmethyl)-2-cyanoiminoimidazolidin,
1-(2-Chloro-5-thiazolylmethyl)-2-cyanoiminotetrahydropyrimidin,
1-(2-Methyl-5-pyrazinylmethyl)-2-cyanoiminoimidazolidin,
1-(2-Chloro-5-pyridylmethyl)-2-cyanoiminothiazolidin,
1-(2-Chloro-5-pyridylmethyl)-2-cyanoiminotetrahydro-2H-1,3-thiazin,
1-(2-Chloro-5-thiazolylmethyl)-2-cyanoiminothiazolidin,
1-(2-Methyl-5-pyrazinylmethyl)-2-cyanoiminothiazolidin,
1-(2-Methyl-5-thiazolylmethyl)-2-cyanoiminothiazolidin
und
1-(1,2,5-Thiadiazol-3-yl)-2-cyanoiminothiazolidin.

Wenn in dem Verfahren a) zur Herstellung der Verbindung der Formel (I) 2-Nitroiminothiazolidin und 2-Chloro-5-pyridylmethylchlorid als Ausgangsstoffe eingesetzt werden, wird die Reaktion durch das folgende Reaktionsschema dargestellt:

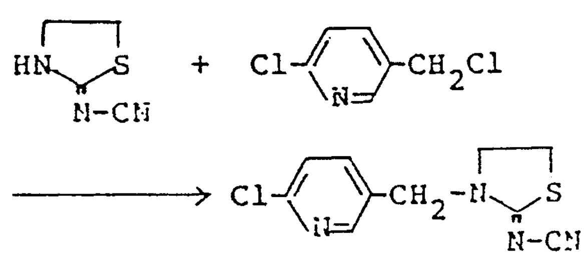

Wenn N-(2-Chloro-5-pyridylmethyl)ethylendiamin und Dimethylcyanodithioimidcarbonat als Ausgangsstoffe in dem Verfahren b) zur Herstellung der Verbindung der Formel (I) eingesetzt werden, wird die Reaktion durch das folgende Reaktionsschema dargestellt:

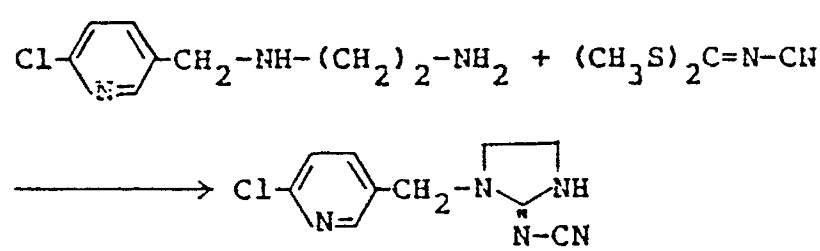

Wenn 1-(2-Chloro-5-pyridylmethyl)-2-cyanoiminoimidazolidin und Acetylchlorid als Ausgangsstoffe in dem Verfahren c) zur Herstellung der Verbindung der Formel (I) eingesetzt werden, wird die Reaktion durch das folgende Reaktionsschema dargestellt:

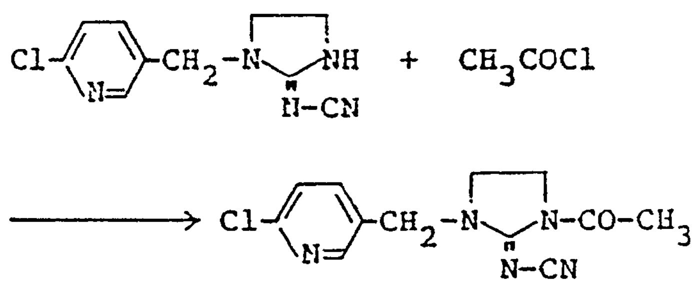

In dem Verfahren a) wird als Ausgangsverbindung der Formel (11) wie vorne definiert, eingesetzt.

Die Verbindungen der Formel (II) umfassen auch bekannte Verbindungen.

2-Cyanoiminoimidazolidin und 2-Cyanoiminotetrahydropyridin sind in J. Org. Chem., Band 38, Seiten 155-156, beschrieben und sind leicht durch die Reaktion von Dimethylcyanodithioimidocarbonat mit Ethylendiamin oder Trimethylendiamin erhältlich. In gleicher Weise liefert die Umsetzung mit Ethylendiamin oder Trimethylendiamin, die mit anderen Substituenten als Acyl N-substituiert sind, das entsprechende 3-substituierte 2-Cyanoiminoimidazolidin oder 3-substituierte 2-Cyanoiminotetrahydropyrimidin.

Bei Einsatz von Aminoalkoholen an Stelle der Alkylendiamine erhält man die entsprechenden Oxazolidine oder Oxazin-Derivate (JP-OS 91064/1973).

2-Cyanoiminothiazolidin ist in Arch. Pharm., Band 305, Seiten 731-737, beschrieben. In gleicher Weise ergibt die Reaktion von 2-Aminopropanthiol mit Dimethylcyanodithioimidocarbonat 2-Cyanoiminotetrahydro-1,3-thiazin.

2-Cyanoiminopyrrolidin ist in Khim. Farm. Zh., Band 19, Seiten 154-158, beschrieben und kann leicht durch Reaktion von 2-Methoxypyrrolin-2 und Cyanamid erhalten werden. In ähnlicher Weise wird 2-Cyanoiminopiperidin aus 2-Methoxy-3,4,5,6-tetrahydropyridin mit Cyanamid erhalten.

In gleicher Weise sind als Ausgangsverbindungen der Formel (III) solche auf der Grundlage der obigen Definitionen für $R^1$, Z und $M^1$ zu verstehen. $M^1$ steht insgesondere für ein Chlor- oder Brom-Atom.

Die Verbindungen der Formel (III) sind in den Japanischen Patentanmeldungen 18627/1985, 18628/1985 und 106853/1985, die von der Anmelderin der vorliegenden Erfindung eingereicht wurden, beschrieben. Zu speziellen Beispielen dieser vorgenannten Anmeldungen zählen
2-Fluoro-5-pyridylmethylchlorid,
2-Chloro-5-pyridylmethylchlorid,
2-Bromo-5-pyridylmethylchlorid,
2-Methyl-5-pyridylmethylchlorid,
2-Chloro-5-thiazolylmethylchlorid,
2-Methyl-5-pyrazinylmethylchlorid,
2-Methyl-5-oxazolylmethylchlorid,
1,2,5-Thiadiazol-3-ylmethylchlorid,
3-Methyl-5-isoxazolylmethylchlorid und
2-Chloro-5-pyrimidinylmethylchlorid.

In dem Verfahren b) sind als Ausgangsverbindungen der Formel (IV) solche auf der Grundlage der Definitionen für $R^1$, A und Z zu verstehen.

Die Verbindungen der Formel (IV) sind in den Japanischen Patentanmeldungen 18627/1985, 18628/1985, 23683/1985, 106853/1985 und 219082/1985 beschrieben. Zu speziellen Beispielen zählen
N-(2-Chloro-5-pyridylmethyl)ethylendiamin oder -trimethylendiamin,
N-(2-Fluoro-5-pyridylmethyl)ethylendiamin oder -trimethylendiamin,
N-(2-Methyl-5-pyridylmethyl)ethylendiamin oder -trimethylendiamin,
N-(2-Methyl-5-thiazolylmethyl)ethylendiamin oder -trimethylendiamin und
N-(2-Methyl-5-pyrazinylmethyl)ethylendiamin oder -trimethylendiamin.

Andere Beispiele umfassen
2-(2-Chloro-5-pyridylmethyl)aminoethanthiol,
3-(2-Chloro-5-pyridylmethyl)aminopropanthiol,

2-(2-Chloro-5-thiazolylmethyl)aminoethanthiol und
2-(2-Methyl-5-pyrazinylmethyl)aminoethanthiol.

Die Ausgangsverbindungen der Formel (V) sind in J. Org. Chem., Band 32, Seiten 1566-1572, beschrieben.

Beim Verfahren c) werden die Ausgangsverbindungen der Formel (Ib) von den Verbindungen der Formel (I) gemäß der vorliegenden Erfindung umfaßt, die mittels der Verfahren a) oder b) hergestellt werden können.

Die Ausgangsverbindungen der Formel (VI) sind auf dem Gebiet der organischen Chemie wohlbekannt, und spezielle Beispiele dafür umfassen Propionylchlorid, Acetylchlorid, Chloroacetylchlorid, Methylsulfonylchlorid, p-Toluolsulfonsäurechlorid und Methoxycarbonylchlorid.

Bei der praktischen Durchführung des Verfahrens a) werden geeignete Verdünnungsmittel verwendet, wobei praktisch sämtliche inerten organischen Lösungsmittel eingesetzt werden können.

Zu Beispielen für die einsetzbaren Verdünnungsmittel zählen aliphatische, alicyclische oder aromatische (gegebenenfalls chlorierte) Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Di-butylether, Propylenoxid, Dioxan und Tetrahydrofuran; Ketone wie Aceton, Methylethylketon und Methylisobutylketon; Nitrile wie Acetonitril, Propionitril und Acrylnitril; Ester wie Ethylacetat und Amylacetat; Säureamide wie Dimethylformamid und Dimethylacetamid; und Sulfone und Sulfoxide wie Dimethylsulfoxid und Sulfolan.

Die Reaktion des Verfahrens a) kann in Gegenwart einer Base durchgeführt werden. Beispiele für die Base sind Alkalimetallhydride wie Natriumhydrid und Kaliumhydrid und Hydroxide und Carbonate von Alkalimetallen.

Das Verfahren a) kann in einem weiten Temperaturbereich durchgeführt werden, beispielsweise zwischen etwa 0 °C und etwa 100 °C, vorzugsweise zwischen etwa 10 °C und etwa 80 °C. Die Reaktion wird zweckmäßigerweise unter Atmosphärendruck durchgeführt, jedoch kann auch bei vermindertem Druch gearbeitet werden.

Bei der praktischen Durchführung des Verfahrens a) wird beispielsweise 1 Mol der Verbindungen der Formel (II) mit 1 bis etwa 1,2 Mol, vorzugsweise 1 bis etwa 1,1 Mol, der Verbindungen der Formel (III) in einem inerten Lösungsmittel wie Dimethylformamid in Anwesenheit einer Base umgesetzt, wodurch die gewünschte Verbindung der Formel (I) erhalten wird.

Bei der praktischen Durchführung des Verfahrens b) zählen zu den geeigneten Verdünnungsmitteln Wasser und Alkohole zusätzlich zu den für das Verfahren a) genannten Lösungsmitteln.

Das Verfahren b) kann in einem weiten Temperaturbereich durchgeführt werden, beispielsweise zwischen 0 °C und dem Siedepunkt der Reaktionsmischung, vorzugsweise zwischen etwa 0 °C und etwa 100 °C. Vorzugsweise wird die Reaktion unter Atmosphärendruck durchgeführt, jedoch kann sie auch unter erhöhtem oder vermindertem Druck durchgeführt werden.

Bei der praktischen Durchführung des Verfahrens b) wird beispielsweise 1 Mol der Verbindung der Formel (IV) mit 1 bis etwa 1,2 Mol vorzugsweise 1 bis etwa 1,1 Mol, der

Verbindungen der Formel (V) in einem inerten Lösungsmittel wie einem Alkohol (z.B. Methanol oder Ethanol) umgesetzt, bis die Mercaptan-Entwicklung aufhört, wodurch die gewünschte neue Verbindung der Formel (I) erhalten wird.

Bei der praktischen Durchführung des Verfahrens c) können als geeignete Verdünnungsmittel die gleichen wie die oben für Verfahren a) genannten eingesetzt werden. Verfahren c) kann in Gegenwart einer Base durchgeführt werden. Die gleichen Alkalimetallhydride, wie sie oben für Verfahren a) genannt wurden, seien als Beispiele für die Basen erwähnt.

Das Verfahren c) kann in einem weiten Temperaturbereich durchgeführt werden, beispielsweise zwischen 0 °C und dem Siedepunkt der Mischung, vorzugsweise zwischen 0 °C und 100 °C. Zweckmäßigerweise wird die Reaktion unter Atmosphärendruck durchgeführt, jedoch kann sie auch unter Bedingungen erhöhten oder verminderten Drucks durchgeführt werden.

Die Verbindungen der Formel (I) gemäß der vorliegenden Erfindung können in Form von Salzen vorliegen, etwa als Salze anorganischer Säuren, Sulfonate, Salze organischer Säuren und Metall-Salze. Dementsprechend sind als neue heterocyclische Verbindungen der Formel (I) in der vorliegenden Erfindung ebenso auch die Salze dieser Verbindungen zu verstehen.

Die aktiven Verbindungen werden von Pflanzen gut vertragen, haben einen günstigen Wert der Toxizität gegenüber warmblütigen Tieren und lassen sich einsetzen zur Bekämpfung von Arthropoden-Schädlingen, insbesondere von Insekten, die in der Land- und Forstwirtschaft auftreten, zum Schutz von Lagerprodukten und Materialien und auf dem Gebiet der Hygiene. Sie sind gegenüber normal empfindlichen und resistenten Species sowie gegen alle oder einige Entwicklungsstadien aktiv. Zu den oben genannten Schädlingen

zählen:

Aus der Klasse der Isopoda beispielsweise
Oniscus asellus,
Armadillidium vulgare und
Porcellio scaber;

aus der Klasse der Diplopoda beispielsweise
Blaniulus guttulatus;

aus der Klasse der Chilopoda beispielsweise
Geophilus carpophagus und
Scutigera spec.;

aus der Klasse der Symphyla beispielsweise
Scutigerella immaculata;

aus der Ordnung der Thysanura beispielsweise
Lepisma saccharina;

aus der Ordnung der Collembola beispielsweise
Onychiurus armatus;

aus der Ordnung der Orthoptera beispielsweise
Blatta orientalis,
Periplaneta americana,
Leucophaea maderae,
Blatella germanica,
Acheta domesticus,
Gryllotalpa spp.,
Locusta migratoria migratorioides,
Melanoplus differentialis und
Schistocerca gregaria;

aus der Ordnung der Dermaptera beispielsweise
Forficula auricularia,

aus der Ordnung der Isoptera beispielsweise
Reticulitermes spp.;

aus der Ordnung der Anoplura beispielsweise
Phylloxera vastatrix,
Pemphigus spp.,
Pediculus humanus corporis,
Haematopinus spp. und
Linognathus spp.;

aus der Ordnung der Mallophaga beispielsweise
Trichodectes spp. und
Damalinea spp.;

aus der Ordnung der Thysanoptera beispielsweise
Hercinothrips femoralis und
Thrips tabaci;

aus der Ordnung der Heteroptera beispielsweise
Eurygaster spp.,
Dysdercus intermedius,
Piesma quadrata,
Cimex lectularius,
Rhodnius prolixus und
Triatoma spp.;

aus der Ordnung der Homoptera beispielsweise
Aleurodes brassicae,
Bemisia tabaci,
Trialeurodes vaporariorum,
Aphis gossypii,
Brevicoryne brassicae,
Cryptomyzus ribis,
Aphis fabae,
Doralis pomi,

Eriosoma lanigerum,
Hyalopterus arundinis,
Macrosiphum avenae,
Myzus spp.,
Phorodon humuli,
Rhopalosiphum padi,
Empoasca spp.,
Euscelis bilobatus,
Nephotettix cincticeps,
Lecanium corni,
Saissetia oleae,
Laodelphax striatellus,
Nilaparvata lugens,
Aonidiella aurantii,
Aspidiotus hederae,
Pseudococcus spp. und
Psylla spp.;

aus der Ordnung der Lepidoptera beispielsweise
Pectinophora gossypiella,
Bupalus piniarius,
Cheimatobia brumata,
Lithocolletis blancardella,
Hyponomeuta padella,
Plutella maculipennis,
Malacosoma neustria,
Euproctis chrysorrhoea,
Lymantria spp.,
Bucculatrix thurberiella,
Phyllocnistis citrella,
Agrotis spp.,
Euxoa spp.,
Feltia spp.,
Earias insulana,
Heliothis spp.,
Spodoptera exigua,
Mamestra brassicae,
Panolis flammea,
Prodenia litura,
Spodoptera spp.,
Trichoplusia ni,
Carpocapsa pomonella,
Pieris spp.,
Chilo spp.,
Pyrausta nubilalis,
Ephestia kuehniella,
Galleria mellonella,
Cacoecia podana,
Capua reticulana,
Choristoneura fumiferana,
Clysia ambiguella,
Homona magnanima und
Tortrix viridana;

aus der Ordnung der Coleoptera beispielsweise
Anobium punctatum,
Rhizopertha dominica,
Acanthoscelides obtectus,
Hylotrupes bajulus,
Agelastica alni,

Leptinotarsa decemlineata,
Phaedon cochleariae,
Diabrotica spp.,
Psylliodes chrysocephala,
Epilachna varivestis,
Atomaria spp.,
Oryzaephilus surinamensis,
Anthonomus spp.,
Sitophilus spp.,
Otiorrhynchus sulcatus,
Cosmopolites sordidus,
Ceuthorrhynchus assimilis,
Hypera postica,
Dermestes spp.,
Trogoderma spp.,
Anthrenus spp.,
Attagenus spp.,
Lyctus spp.,
Meligethes aeneus,
Ptinus spp.,
Niptus hololeucus,
Gibbium psylloides,
Tribolium spp.,
Tenebrio molitor,
Agriotes spp.,
Conoderus spp.,
Melolontha melolontha,
Amphimallon solstitialis und
Costelytra zealandica;

aus der Ordnung der Hymenoptera beispielsweise
Diprion spp.,
Hoplocampa spp.,
Lasius spp.,
Monomorium pharaonis und
Vespa spp.;

aus der Ordnung der Diptera beispielsweise
Aedes spp.,
Anopheles spp.,
Culex spp.,
Drosophila melanogaster,
Musca spp.,
Fannia spp.,
Calliphora erythrocephala,
Lucilia spp.,
Chrysomyia spp.,
Cuterebra spp.,
Gastrophilus spp.,
Hyppobosca spp.,
Stomoxys spp.,
Oestrus spp.,
Hypoderma spp.,
Tabanus spp.,
Tannia spp.,
Bibio hortulanus,
Oscinella frit,
Phorbia spp.,
Pegomyia hyoscami,
Ceratitis capitata,

Dacus oleae und
Tipula paludosa.

Auf dem Gebiet der Veterinärmedizin sind die neuen Verbindungen der vorliegenden Erfindung gegen verschiedene schädliche Tierparasiten (innere und äußere Parasiten) wie Insekten und Würmer wirksam.

Beispielen für solche Tierparasiten sind Insekten wie
Gastrophilus spp.,
Stomoxys spp.,
Trichodectes spp.
Rhodnius spp. und
Ctenocephalidex canis.

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung ebenfalls verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die aktiven Verbindungen gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Es ist jedoch ausdrücklich darauf hinzuweisen, daß die vorliegende Erfindung in keiner Weise allein auf diese Beispiele beschränkt ist.

Herstellungsbeispiele

## Beispiel 1

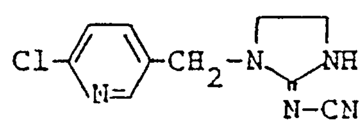

(Verbindung Nr. 5)

N-(2-Chloro-5-pyridylmethyl)ethylendiamin (3,7 g) und Dimethylcyanodithioimidocarbonat (1,3 g) wurden zu 50 ml Ethanol hinzugefügt, und die Mischung wurde allmählich unter Rühren erwärmt und anschließend 3 Stunden zum Rückfluß erhitzt. Nach der Reaktion wurde das Ethanol unter vermindertem Druck abdestilliert, wonach der Rückstand erstarrte. Der erstarrte Rückstand wurde pulverisiert und mit einer Mischung aus Ether und einer kleinen Menge Ethanol gewaschen. Die Menge des nach dem Trocknen erhaltenen Produkts betrug 3,5 g; Schmp. 167-170 °C.

## Beispiel 2

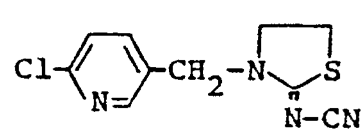

(Verbindung Nr. 64

N-(2-Chloro-5-pyridylmethyl)cysteamin (2,0 g) und Dimethylcyanodithioimidocarbonat (1,3 g) wurden zu 50 ml Ethanol hinzugefügt. Im Stickstoff-Gasstrom wurde die Mischung 8 h unter Rühren zum Rückfluß erhitzt. Nach der Reaktion wurden etwa 2/3 des Ethanols unter vermindertem Druck abdestilliert. Beim Stehenlassen des Rückstandes bei Raumtemperatur fiel das Endprodukt in Form von Kristallen aus. Die

Kristalle wurden durch Filtration gesammelt, mit Ether gewaschen und getrocknet. Die Menge des erhaltenen Produkts betrug 2,4 g; Schmp. 128-129 °C.

## Beispiel 3

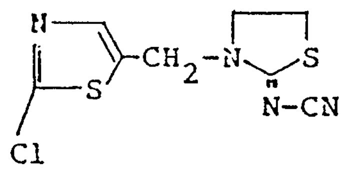

(Verbindung Nr. 68

Eine Mischung aus 2-Cyanoiminothiazolidin (2,5 g), wasserfreiem Kaliumcarbonat (3,0 g), 2-Chloro-5-chloromethylthiazol (3,3 g) und trockenem Acetonitril wurde unter gutem Rühren 3 h zum Rückfluß erhitzt. Nach der Reaktion wurde das Acetonitril unter vermindertem Druck abdestilliert, und zu dem Rückstand wurde Dichloromethan hinzugefügt. Die Mischung wurde mit Wasser und mit einer 1-proz. wäßrigen Natriumhydroxid-Lösung gewaschen. Die Dichloromethan-Schicht wurde getrocknet und konzentriert. Der Niederschlag wurde durch Filtration gesammelt und getrocknet. Die Menge des erhaltenen Produkts betrug 3,3 g; Schmp. 145-146 °C.

## Beispiel 4

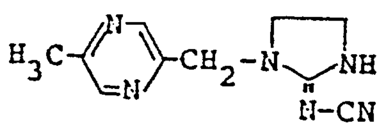

(Verbindung Nr. 54)

2-Cyanoiminoimidazolidin (2,2 g) wurde in 25 ml trockenem Dimethylformamid gelöst, und Natriumhydrid (1 g) wurde in kleinen Portionen bei weniger als 10 °C hinzugefügt, und die Mischung wurde bei 10 °C gerührt, bis die Wasserstoff-Entwicklung beendet war. Dann wurde eine Lösung von 2-Chloromethyl-5-methylpyrazin (2,8 g) in Dimethylformamid (10 ml) tropfenweise bei 10 °C zugegeben. Nach der Zugabe wurde die Mischung 1 Stunde bei Raumtemperatur gerührt. Eiswasser wurde zu der Mischung hinzugefügt, und der pH der wäßrigen Lösung wurde auf 7 eingestellt. Die wäßrige Schicht wurde mit Dichloromethan extrahiert, und die Dichloromethan-Schicht wurde mit Wasser gewaschen und getrocknet. Nach dem Konzentrieren des Dichloromethans wurde der verbleibende Feststoff aus verdünntem Ethanol umkristallisiert, wonach 1,8 g des Endprodukts erhalten wurden; Schmp. 144-147 °C.

## Beispiel 5

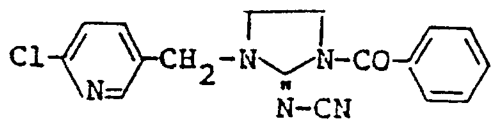

(Verbindung Nr. 87

1-(2-Chloro-5-pyridylmethyl)-2-cyanoiminoimidazolidin (2,4 g) wurde in 30 ml trockenem Dimethylformamid gelöst, und Natriumhydrid (0,26 g) wurde bei 10 °C hinzugefügt. Die Mischung wurde bei Raumtemperatur gerührt, bis die Wasserstoff-Entwicklung beendet war. Dann wurde Benzoylchlorid (1,4 g) hinzugegeben, und die Mischung wurde 30 min bei 40 °C gerührt und dann in Eiswasser gegossen. Die wäßrige Schicht wurde mit Dichloromethan extrahiert. Die Dichloromethan-Schicht wurde mit Wasser gewaschen, und das Dichloromethan wurde konzentriert. Der Rückstand wurde durch Silicagel-Säulenchromatographie gereinigt, wonach das Endprodukt erhalten wurde. Dessen Menge betrug 1,3 g; Schmp. 158-161 °C.

Die in der Tabelle 1 aufgeführten Verbindungen können in der gleichen Weise hergestellt werden, wie sie in den Beispielen 1 bis 5 beispielhaft dargestellt ist. Tabelle 1 offenbart auch die in den Beispielen 1 bis 5 erhaltenen Verbindungen.

Tabelle 1

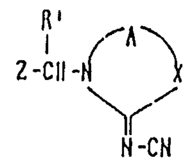

| Verbindung Nr. | R'<br>Z-CH- | A | X | physikalische Stoff-charakteristika |
|---|---|---|---|---|
| 1 | 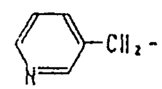 | $-CH_2CH_2-$ | NH | |
| 2 | 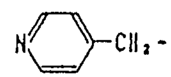 | $-CH_2CH_2-$ | NH | Schmp. 191 ~ 193°C |
| 3 | 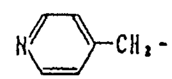 | $-(CH_2)_3-$ | NH | Schmp. 214 ~ 216°C |
| 4 | 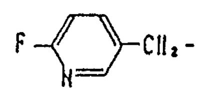 | $-CH_2CH_2-$ | NH | Schmp. 154 ~ 157°C |
| 5 | 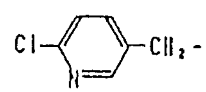 | $-CH_2CH_2-$ | NH | Schmp. 167 ~ 170°C |
| 6 | 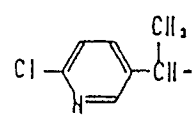 | $-CH_2CH_2-$ | NH | |

| | | | | |
|---|---|---|---|---|
| 7 | 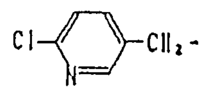 | 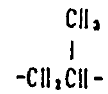 | NH | Schmp. 155~160°C |
| 8 | 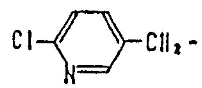 | $-(CH_2)_3-$ | NH | Schmp. 166~167.5°C |
| 9 | 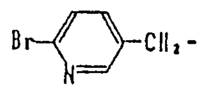 | $-CH_2CH_2-$ | NH | Schmp. 174~178°C |
| 10 | 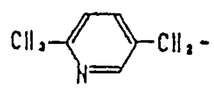 | $-CH_2CH_2-$ | NH | |
| 11 | 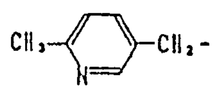 | $-(CH_2)_3-$ | NH | Schmp. 184~188°C |
| 12 | 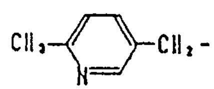 | 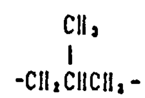 | NH | |
| 13 | 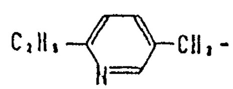 | $-CH_2CH_2-$ | NH | |

| | | | | |
|---|---|---|---|---|
| 14 | 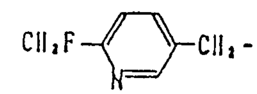 | $-CH_2CH_2-$ | NH | |
| 15 | 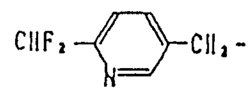 | $-CH_2CH_2-$ | NH | |
| 16 | 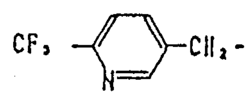 | $-CH_2CH_2-$ | NH | |
| 17 | 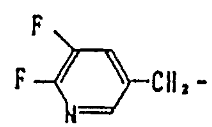 | $-CH_2CH_2-$ | NH | |
| 18 | 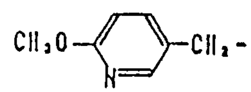 | $-CH_2CH_2-$ | NH | |
| 19 | 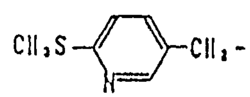 | $-(CH_2)_3-$ | NH | |
| 20 | 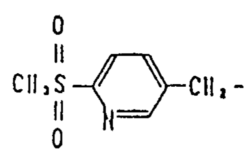 | $-CH_2CH_2-$ | NH | |

| | | | | |
|---|---|---|---|---|
| 21 | 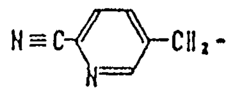 | $-CH_2CH_2-$ | NH | |
| 22 | 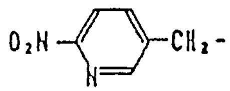 | $-CH_2CH_2-$ | NH | |
| 23 | 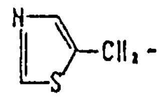 | $-(CH_2)_3-$ | NH | |
| 24 | 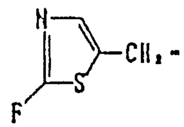 | $-(CH_2)_3-$ | NH | |
| 25 | 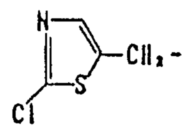 | $-CH_2CH_2-$ | NH | Schmp. 181~182℃ |
| 26 | 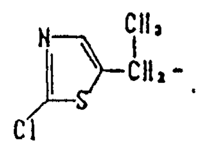 | $-CH_2CH_2-$ | NH | |
| 27 | 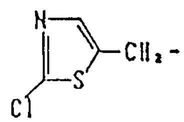 | $-(CH_2)_3-$ | NH | Schmp. 182~185℃ |
| 28 | 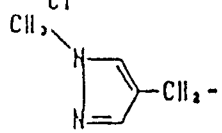 | $-(CH_2)_3-$ | NH | |

| | | | | |
|---|---|---|---|---|
| 29 | 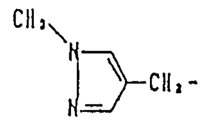 | $-CH_2C(CH_3)_2-CH_2-$ | NH | Schmp. 224~227°C |
| 30 | 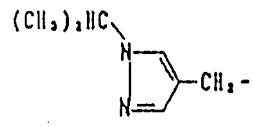 | $-CH_2CH_2-$ | NH | |
| 31 | 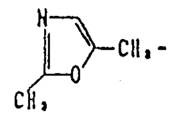 | $-CH_2CH_2-$ | NH | |
| 32 | 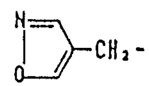 | $-CH_2CH_2-$ | NH | |
| 33 | 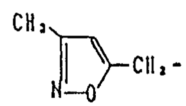 | $-(CH_2)_3-$ | NH | Schmp. 145~148°C |
| 34 | 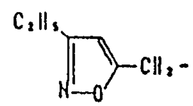 | $-CH_2CH_2-$ | NH | Schmp. 129~131°C |
| 35 | 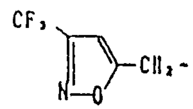 | $-(CH_2)_3-$ | NH | |
| 36 | 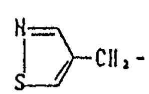 | $-CH_2CH_2-$ | NH | |

| | | | | |
|---|---|---|---|---|
| 37 | 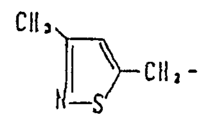 | $-(CH_2)_3-$ | NH | |
| 38 | 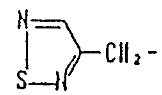 | $-CH_2CH_2-$ | NH | |
| 39 | 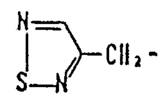 | $-(CH_2)_3-$ | NH | Schmp. 137 ~ 140°C |
| 40 | 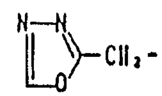 | $-(CH_2)_3-$ | NH | |
| 41 | 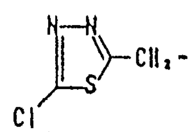 | $-(CH_2)_3-$ | NH | |
| 42 | 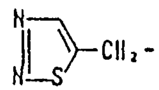 | $-CH_2CH_2-$ | NH | |
| 43 | 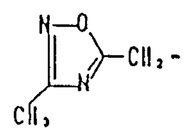 | $-(CH_2)_3-$ | NH | |
| 44 | 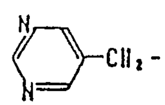 | $-CH_2CH_2-$ | NH | |

| | | | | |
|---|---|---|---|---|
| 45 | 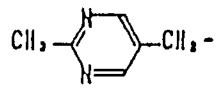 | $-CH_2CH_2-$ | NH | |
| 46 | 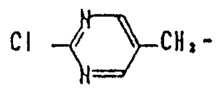 | $-CH_2CH_2-$ | NH | |
| 47 | 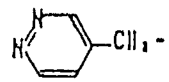 | $-CH_2CH_2-$ | NH | |
| 48 | 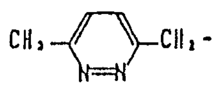 | $-(CH_2)_3-$ | NH | |
| 49 | 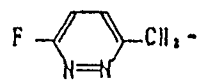 | $-CH_2CH_2-$ | NH | |
| 50 | 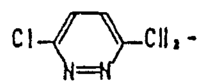 | $-CH_2CH_2-$ | NH | |
| 51 | 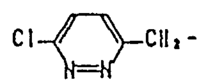 | $-(CH_2)_3-$ | NH | Schmp. 185~188°C |
| 52 | 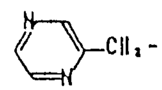 | $-CH_2CH_2-$ | NH | |
| 53 | 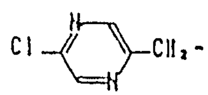 | $-CH_2CH_2-$ | NH | |
| 54 | 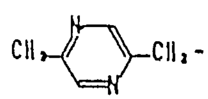 | $-CH_2CH_2-$ | NH | Schmp. 144~147°C |

| | | | | |
|---|---|---|---|---|
| 55 | 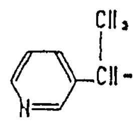 | $-CH_2CH_2-$ | N-CHO | |
| 56 | 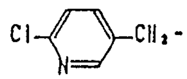 | $-CH_2CH_2-$ | $N-COCH_3$ | $n_D^{20}$ 1.5895 |
| 57 | 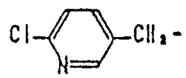 | $-CH_2CH_2-$ | $N-COCH_2Cl$ | Schmp. 53~55℃ |
| 58 | 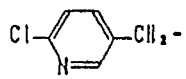 | $-CH_2CH_2-$ | $N-COC(CH_3)_3$ | |
| 59 | 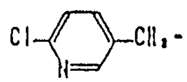 | $-CH_2CH_2-$ | 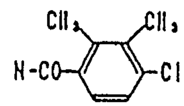 | |
| 60 | 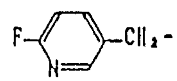 | $-(CH_2)_3-$ | 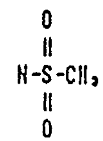 | |
| 61 | 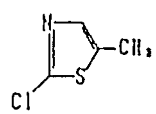 | $-(CH_2)_3-$ | $N-COOC_2H_5$ | |
| 62 | 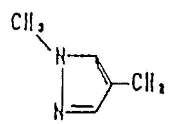 | $-CH_2CH_2-$ | 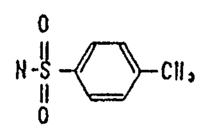 | |

| | | | | |
|---|---|---|---|---|
| 63 | 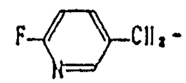 | $-CH_2CH_2-$ | S | Schmp. 117~120℃ |
| 64 | 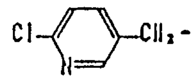 | $-CH_2CH_2-$ | S | Schmp. 128~129℃ |
| 65 | 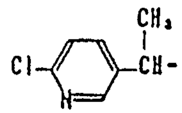 | $-CH_2CH_2-$ | S | |
| 66 | 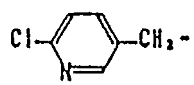 | $-(CH_2)_3-$ | S | Schmp. 124~125℃ |
| 67 | 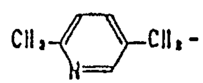 | $-CH_2CH_2-$ | S | |
| 68 | 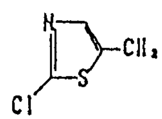 | $-CH_2CH_2-$ | S | Schmp. 145~146℃ |
| 69 | 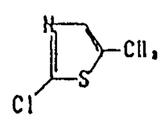 | $-(CH_2)_3-$ | S | |
| 70 | 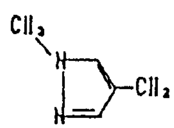 | $-(CH_2)_3-$ | S | |

| | | | | |
|---|---|---|---|---|
| 71 | 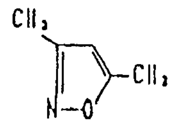 | $-(CH_2)_3-$ | S | |
| 72 | 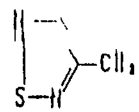 | $-CH_2CH_2-$ | S | Schmp. 153 - 157°C |
| 73 | 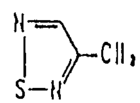 | $-(CH_2)_3-$ | S | |
| 74 | 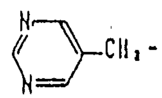 | $-CH_2CH_2-$ | S | |
| 75 | 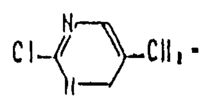 | $-CH_2CH_2-$ | S | |
| 76 | 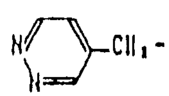 | $-CH_2CH_2-$ | S | |
| 77 | 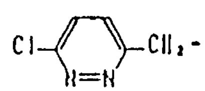 | $-(CH_2)_3-$ | S | |
| 78 | 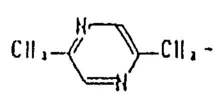 | $-CH_2CH_2-$ | S | Schmp.132 ~ 135°C |

| | | | | |
|---|---|---|---|---|
| 79 | 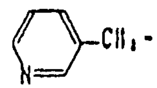 | $-CH_2CH_2-$ | 0 | |
| 80 | 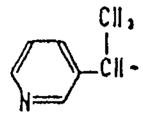 | $-CH_2CH_2-$ | 0 | |
| 81 | 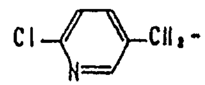 | $-CH_2CH_2-$ | 0 | Schmp. 113~114°C |
| 82 | 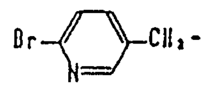 | $-(CH_2)_3-$ | 0 | |
| 83 | 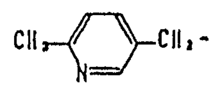 | $-CH_2CH_2-$ | 0 | |
| 84 | 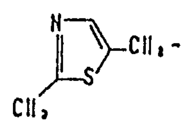 | $-CH_2CH_2-$ | 0 | |
| 85 | 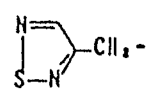 | $-(CH_2)_3-$ | 0 | |
| 86 | 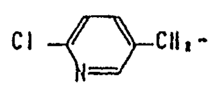 | $-CH_2CH_2-$ | $N-COCCl_3$ | |
| 87 | 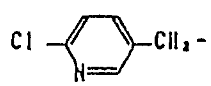 | $-CH_2CH_2-$ | N-CO- 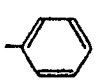 | Schmp. 158~161°C |

| | | | | |
|---|---|---|---|---|
| 88 | 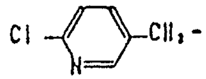 | $-CH_2CH_2-$ | $N-COOCH_3$ | |
| 89 | 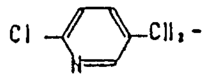 | $-CH_2CH_2-$ | N-COO-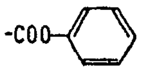 | |
| 90 | 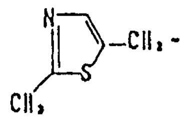 | $-CH_2CH_2-$ | $N-COCH_3$ | |
| 91 | 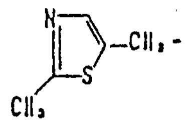 | $-CH_2CH_2-$ | $N-COOCH_3$ | |
| 92 | 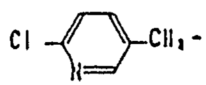 | $-(CH_2)_3-$ | $N-COSC_2H_5$ | |
| 93 | 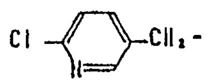 | $-(CH_2)_3-$ | $N-SO_2-CH_2Cl$ | |
| 94 | 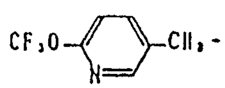 | $-CH_2CH_2-$ | $N-COC_2H_5$ | |
| 95 | 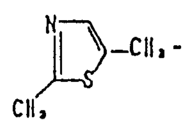 | $-CH_2CH_2-$ | S | Schmp. 138 ~ 140°C |

| | | | | |
|---|---|---|---|---|
| 96 | 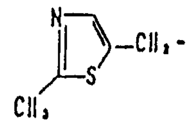 | $-(CH_2)_3-$ | S | |
| 97 | 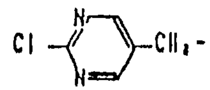 | $-CH_2CH_2-$ | 0 | |
| 98 | 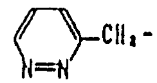 | $-CH_2CH_2-$ | 0 | |
| 99 | 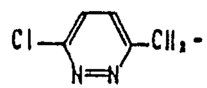 | $-CH_2CH_2-$ | 0 | |
| 100 | 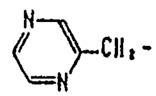 | $-(CH_2)_3-$ | 0 | |
| 101 | 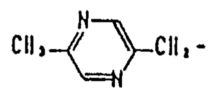 | $-CH_2CH_2-$ | 0 | Schmp. 130 ~ 134°C |
| 102 | 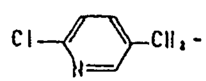 | $-CH_2CH_2-$ | $CH_2$ | |
| 103 | 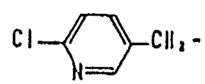 | $-CH_2CH_2-$ | $CH-CH_3$ | |

| | | | | |
|---|---|---|---|---|
| 104 | 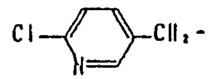 | $-(CH_2)_3-$ | $CH_2$ | Schmp. 74 ~ 78°C |
| 105 | 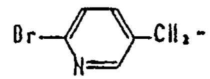 | $-CH_2CH_2-$ | $CH_2$ | |
| 106 | 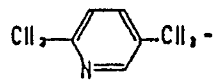 | $-CH_2CH_2-$ | $CH_2$ | |
| 107 | 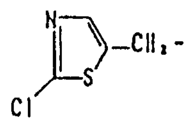 | $-CH_2CH_2-$ | $CH_2$ | |
| 108 | 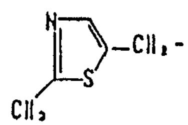 | $-(CH_2)_3-$ | $CH_2$ | Schmp. 122 ~ 125°C |
| 109 | 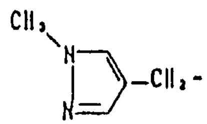 | $-(CH_2)_3-$ | $CH_2$ | |
| 110 | 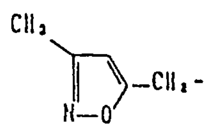 | $-CH_2CH_2-$ | $CH_2$ | |
| 111 | 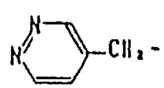 | $-CH_2CH_2-$ | O | |

| | | | | |
|---|---|---|---|---|
| 112 | 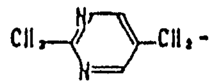 | $-CH_2CH_2-$ | O | |
| 113 | 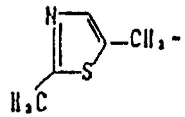 | $-(CH_2)_3-$ | NH | Schmp. 185 ~ 190 ℃ |
| 114 | 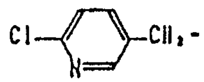 | $-CH_2CH_2-$ | $N-CH_3$ | Schmp. 101 ~ 103 ℃ |
| 115 | 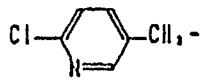 | $-CH_2CH_2-$ | $N-CH_2CN$ | $n_D^{19}$ 1.6015 |
| 116 | 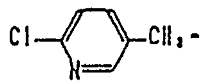 | $-CH_2CH_2-$ | $N-CH_2CF_3$ | |
| 117 | 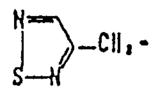 | $-(CH_2)_3-$ | $N-CH_2CH_2OCH_3$ | |
| 118 | 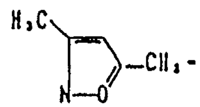 | $-CH_2CH_2-$ | $N-CH_2CH_2SC_2H_5$ | |
| 119 | 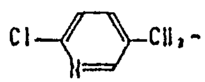 | $-CH_2CH_2-$ | $N-CH_2-$ 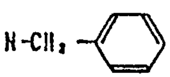 | $n_D^{19}$ 1.6145 |
| 120 | 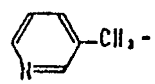 | $-CH_2CH_2-$ | $N-CH_2-$ 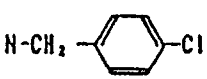 | |

| | | | | |
|---|---|---|---|---|
| 121 | 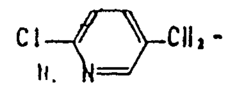 | $-CH_2CH_2-$ | $N-CH_2CH=CH_2$ | |
| 122 | 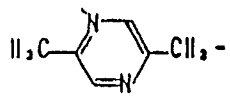 | $-CH_2CH_2-$ | $N-CH_2C\equiv CH$ | |
| 123 | 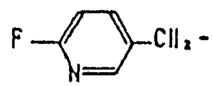 | $-CH_2CH_2-$ | $N-CH_2CH_2CN$ | |
| 124 | 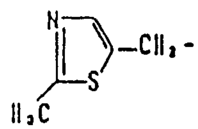 | $-(CH_2)_3-$ | S | Schmp. 141 ~ 145°C |
| 125 | 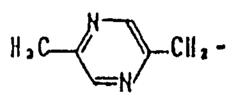 | $-CH_2CH_2-$ | $CH_2$ | Schmp. 85 ~ 90°C |
| 126 | 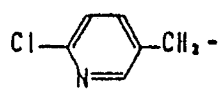 | $-CH_2CH_2-$ | 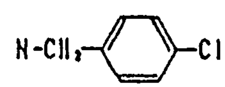 | Schmp. 181 ~ 183°C |
| 127 | 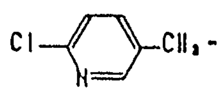 | $-CH_2CH_2-$ | $N-COOC_2H_5$ | $n_D^{19}$ 1.5880 |

| | | | | |
|---|---|---|---|---|
| 128 | 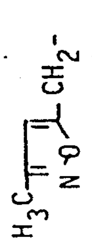 | $-(CH_2)_3-$ | $N-CH_2C\equiv CH$ | $n_D^{20}$ 1.5667 |
| 129 | 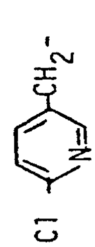 | $-CH_2-CH_2-$ | $N-CH_2\overset{O}{\overset{\|\|}{C}}C(CH_3)_3$ | $n_D^{20}$ 1.5446 |
| 130 | 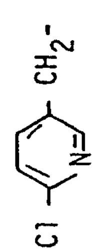 | $-CH_2-\underset{}{CH}(CH_3)-$ | O | Schmp. 119 - 121°C |

Verwendungsbeispiele

Vergleichsverbindungen des nächstliegenden Standes der Technik:

W-1:

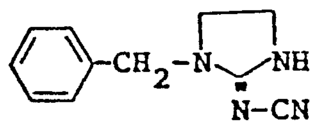

(beschrieben in der JP-OS 91064).

W-2:

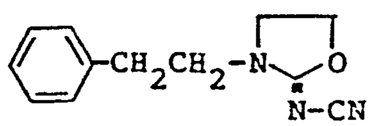

(beschrieben in dem oben zitierten Patent-Dokument).

Q-1:

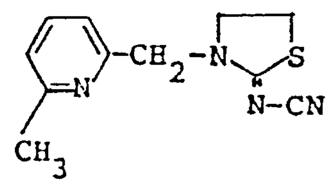

(beschrieben in der JP-OS 196877/1984).

Beispiel 6 (Biologischer Test)

Test mit gegen Organophosphor-Mittel resistenten grünen Reis-Blattwanzen (Nephotettix cincticeps):

Herstellung einer Test-Chemikalie:

| | | |
|---|---|---|
| Lösungsmittel: | Xylol | 3 Gew.-Teile |
| Emulgator: | Polyoxyethylen-alkylphenylether | 1 Gew.-Teil |

Zur Herstellung einer Formulierung eines geeigneten Wirkstoffs wurde 1 Gew.-Teil des Wirkstoffs mit der oben bezeichneten Menge Lösungsmittel, das die oben angegebene Menge Emulgator enthielt, vermischt, und die Mischung wurde mit Wasser auf eine vorher festgesetzte Konzentration verdünnt.

Test-Verfahren:

Eine Wasser-Verdünnung des Wirkstoffs mit einer vorher festgelegten Konzentration, die wie oben beschrieben hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Die aufgesprühte Chemikalie wurde trocknen gelassen, und über jeden der Töpfe wurde ein Drahtkorb von 7 cm Durchmesser und 14 cm Höhe gestülpt, unter dem 30 ausgewachsene weibliche Exemplare der Reis-Kleinzikade eines Stammes, der gegen Organophosphat-Chemikalien Resistenz zeigte, ausgesetzt wurden. Die Töpfe wurden in einem Raum mit konstanter Temperatur aufbewahrt. Zwei Tage später wurde die Zahl der toten Insekten bestimmt, und das Tötungsverhältnis wurde berechnet.

Im Vergleich zu den Vergleichsverbindungen W-1, W-2 und Q-1 zeigten beispielsweise die folgenden Verbindungen gemäß der vorliegenden Erfindung eine beträchtlich bessere Wirksamkeit: Verbindungen Nr. 4, 5, 8, 9, 25, 27, 54, 64, 66, 68, 78.

Beispiel 7 (Biologischer Test)

Test mit Laternenträgern

Test-Verfahren:

Eine Wasser-Verdünnung jeder der Wirkstoffe mit einer vorher festgelegten Konzentration, die wie in dem vorhergehenden Beispiel beschrieben, hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Die aufgesprühte Chemikalie wurde trocknen gelassen, und über jeden der Töpfe wurde ein Drahtkorb von 7 cm Durchmesser und 14 cm Höhe gestülpt, und 30 ausgewachsene weibliche Exemplare des braunen Laternenträgers (Nilaparvata lugens) eines Stammes, der gegen Organophosphat-Chemikalien Resistenz zeigte, wurden unter dem Korb ausgesetzt, und die Töpfe wurden in einem Raum mit konstanter Temperatur aufbewahrt. Zwei Tage später wurde die Zahl der toten Insekten bestimmt, und das Tötungsverhältnis wurde berechnet.

In der gleichen Weise wurde das Tötungsverhältnis für den Weißrücken-Laternenträger (Sogatella furcifera) und den organophosphat-resistenten kleineren braunen Laternenträger (Laodelphax striatellus) berechnet.

Im Vergleich zu den Vergleichsverbindungen W-1, W-2 und Q-1 zeigten beispielsweise die folgenden Verbindungen gemäß der vorliegenden Erfindung eine beträchtlich bessere Wirksamkeit gegen braune Laternenträger, braune kleinere Laternenträger und Weißrücken-Laternenträger: Verbindungen Nr. 4, 5, 8, 9, 25, 27, 64, 66.

Beispiel 8 (Biologischer Test)

Test mit grünen Pfirsichblattläusen (Myzus persicae), die gegen Organophosphat- und Carbamat-Chemikalien Resistenz zeigten:

Test-Verfahren:

Gezüchtete grüne Pfirsichblattläuse, die gegen Organophosphate und Carbamate Resistenz zeigten, wurden auf Auberginen-Setzlingen (braune längliche Varietät) von etwa 20 cm Höhe ausgesetzt, die in unglasierten Töpfen von 15 cm Durchmesser gezogen worden waren, und zwar mit einer Befallsrate von etwa 200 Individuen pro Setzling. Einen Tag nach dem Aussetzen wurde eine Wasser-Verdünnung jeder der aktiven Verbindungen mit einer vorher festgelegten Konzentration, die wie in Beispiel 6 beschrieben hergestellt worden war, in genügenden Mengen mit Hilfe einer Spritzpistole aufgesprüht. Nach dem Sprühen wurden die Töpfe in einem Gewächshaus bei 28 °C stehen gelassen. 24 h nach dem Sprühen wurde das Tötungsverhältnis berechnet. Der vorstehende Test wurde mit zwei Wiederholungen durchgeführt.

Im Vergleich zu den Vergleichsverbindungen W-1, W-2 und Q-1 zeigten beispielsweise die folgenden Verbindungen gemäß der vorliegenden Erfindung eine beträchtlich bessere Wirksamkeit gegen Myzus persicae: Verbindungen Nr. 4, 5, 25, 27, 64, 66, 68.

Die in den Beispielen 6, 7 und 8 dargestellten biologischen Tests sind nur typische Beispiele für die Verwendung der Verbindungen der vorliegenden Erfindung als Insektizide. Die hierin gezeigten Verbindungen der vorliegenden Erfindung sind typische Beispiele, und der Nutzwert der Erfindung ist nicht allein auf diese Beispiele beschränkt.

**Patentansprüche**

1. Heterocyclische Verbindungen der Formel (I)

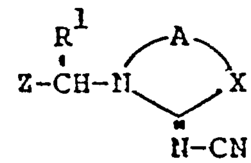

(I)

in welcher

$R^1$ ein Wasserstoff-Atom oder eine Methyl-Gruppe bezeichnet,

A eine Ethylen-Gruppe, die durch Methyl substituiert sein kann, oder eine Trimethylen-Gruppe, die durch Methyl substituiert sein kann, bezeichnet,

X ein Sauerstoff- oder Schwefel-Atom oder die Gruppe

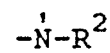

oder

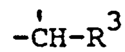

bezeichnet, worin

$R^2$ ein Wasserstoff-Atom, eine $C_1$-$C_4$-Alkyl-Gruppe, die durch einen aus Halogenen, $C_1$-$C_4$-Alkoxy-Gruppen, $C_1$-$C_4$-Alkylthio-Gruppen und Cyano ausgewählten Substituenten substituiert sein kann, eine $C_2$-$C_4$-Alkenyl-Gruppe, eine $C_2$-$C_4$-Alkinyl-Gruppe, eine Pyridylmethyl-Gruppe, die durch Halogen und/oder Methyl substituiert sein kann, eine Benzyl-Gruppe, die durch Halogen und/oder Methyl substituiert sein kann, eine Formyl-Gruppe, eine Alkylcarbonyl-Gruppe mit 1 bis 2-Kohlenstoff-Atomen in der Alkyl-Struktureinheit, die durch Halogen substituiert sein kann, eine Phenylcarbonyl-Gruppe, die durch Halogen und/oder Methyl substituiert sein kann, eine Alkoxy- oder Alkylthiocarbonyl-Gruppe mit 1 bis 4-Kohlenstoff-Atomen in der Alkyl-Struktureinheit, eine Phenoxycarbonyl-Gruppe, eine $C_1$-$C_4$-Alkylsulfonyl-Gruppe, die durch Halogen substituiert sein kann, oder eine Phenylsulfonyl-Gruppe, die durch Methyl substituiert sein kann, bezeichnet und

$R^3$ ein Wasserstoff-Atom bezeichnet, und

Z eine 5- oder 6-gliedrige heterocyclische Gruppe, die zwei oder drei aus Sauerstoff-, Schwefel- und Stickstoff-Atomen ausgewählte Hetero-Atome enthält, von denen wenigstens eines ein Stickstoff-Atom ist, oder eine 3-Pyridyl-Gruppe bezeichnet, wobei die heterocyclische Gruppe und die 3-Pyridyl-Gruppe gegebenenfalls durch wenigstens einen aus Halogen-Atomen, Alkyl-Gruppen mit 1 bis 4 Kohlenstoff-Atomen, Alkoxy-Gruppen mit 1 bis 4 Kohlenstoff-Atomen, Alkylthio-Gruppen mit 1 bis 4 Kohlenstoff-Atomen, Halogenoalkyl-Gruppen mit 1 bis 4 Kohlenstoff-Atomen, Halogenoalkoxy-Gruppen mit 1 bis 4 Kohlenstoff-Atomen, Alkylsulfonyl-Gruppen mit 1 bis 4 Kohlenstoff-Atomen, der Cyano-Gruppe und der Nitro-Gruppe ausgewählten Substituenten substituiert sind,

mit Ausnahme folgender Verbindung:

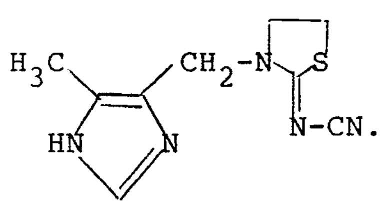

**2.** Verbindungen nach Anspruch 1, dadurch gekennzeichnet, daß

$R^1$ ein Wasserstoff-Atom bezeichnet,

A eine Ethylen- oder Trimethylen-Gruppe bezeichnet,

X ein Schwefel-Atom oder die Gruppe

-NH

bezeichnet und

Z eine 5- oder 6-gliedrige heterocyclische Gruppe, die zwei aus Sauerstoff-, Schwefel- und Stickstoff-Atomen ausgewählte Hetero-Atome enthält, von denen wenigstens eines ein Stickstoff-Atom ist, oder eine 3-Pyridyl-Gruppe bezeichnet, wobei die heterocyclische Gruppe und die 3-Pyridyl-Gruppe gegebenenfalls durch wenigstens einen aus dem Fluor-Atom, dem Chlor-Atom, dem Brom-Atom, der Methyl-Gruppe, der Methoxy-Gruppe, der Methylthio-Gruppe, der Trifluoromethyl-Gruppe, der Trifluoromethoxy-Gruppe, der Methylsulfonyl-Gruppe, der Cyano-Gruppe und der Nitro-Gruppe ausgewählten Substituenten substituiert sind, mit Ausnahme folgender Verbindung:

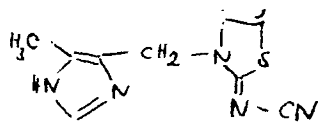
.

**3.** Heterocyclische Verbindungen nach Ansprüchen 1 bis 2 , ausgewählt aus den nachstehenden Verbindungen:

1-(2-Chloro-5-pyridylmethyl)-2-cyanoiminoimidazolidin der Formel

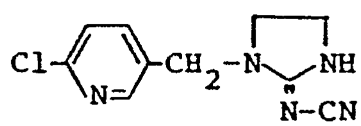
,

1-(2-Fluoro-5-pyridylmethyl)-2-cyanoiminoimidazolidin der Formel

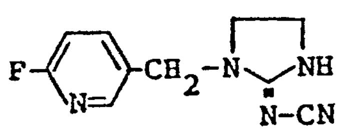
,

1-(2-Chloro-5-pyridylmethyl)-2-cyanoiminotetrahydropyrimidin der Formel

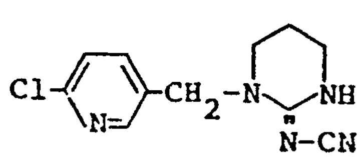
,

1-(2-Methyl-5-pyridylmethyl)-2-cyanoiminoimidazolidin der Formel

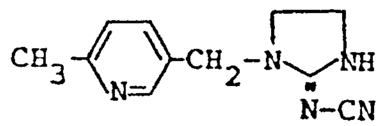

,

1-(2-Chloro-5-thiazolylmethyl)-2-cyanoiminoimidazolidin der Formel

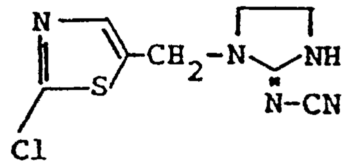

,

1-(2-Chloro-5-thiazolylmethyl)-2-cyanoiminotetrahydropyrimidin der Formel

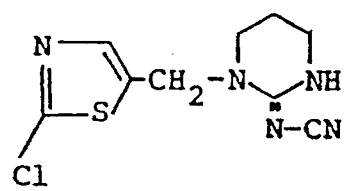

,

1-(2-Methyl-5-pyrazinylmethyl)-2-cyanoiminoimidazolidin der Formel

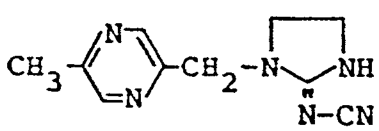

,

1-(2-Chloro-5-pyridylmethyl)-2-cyanoiminothiazolidin der Formel

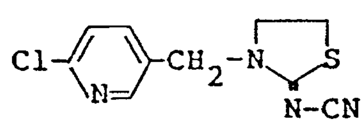

,

1-(2-Chloro-5-pyridylmethyl)-2-cyanoiminotetrahydro-2H-1,3-thiazin der Formel

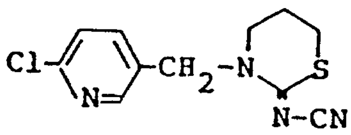

,

1-(2-Chloro-5-thiazolylmethyl)-2-cyanoiminothiazolidin der Formel

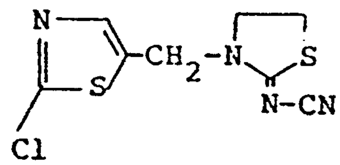

,

1-(2-Methyl-5-pyrazinylmethyl)-2-cyanoiminothiazolidin der Formel

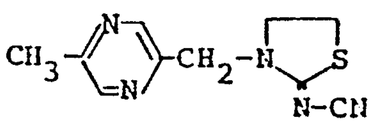

,

1-(2-Methyl-5-thiazolylmethyl)-2-cyanoiminothiazolidin der Formel

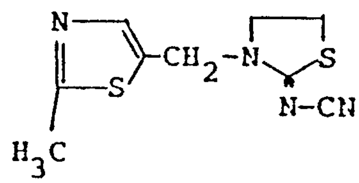

und 1-(1,2,5-Thiadiazol-3-yl)-2-cyanoiminothiazolidin der Formel

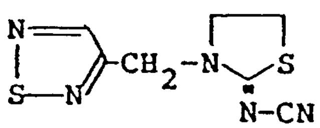

.

**4.** Insektizide Mittel, dadurch gekennzeichnet, daß sie wenigstens eine heterocyclische Verbindung der Formel (I) gemäß Anspruch 1 enthalten.

5. Verfahren zur Bekämpfung schädlicher Insekten, dadurch gekennzeichnet, daß man heterocyclische Verbindungen der Formel (I) gemäß Anspruch 1 auf die schädlichen Insekten und/oder deren Lebensraum einwirken läßt.

6. Verwendung neuer heterocyclischer Verbindungen der Formel (I) gemäß Anspruch 1 zur Bekämpfung schädlicher Insekten.

7. Verfahren zur Herstellung insektizider Mittel, dadurch gekennzeichnet, daß neue heterocyclische Verbindungen der Formel (I) gemäß Anspruch 1 mit Streckmitteln und/oder grenzflächenaktiven Mitteln vermischt werden.

**Claims**

1. Heterocyclic compounds of the formula (I)

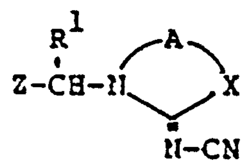

(I)

in which

$R^1$ denotes a hydrogen atom or a methyl group,

A denotes an ethylene group which can be substituted by methyl, or a trimethylene group which can be substituted by methyl,

X denotes an oxygen or sulphur atom or the group

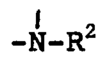

or

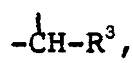

in which

$R^2$ denotes a hydrogen atom, a $C_1$-$C_4$-alkyl group which can be substituted by a substituent selected from amongst halogens, $C_1$-$C_4$-alkoxy groups, $C_1$-$C_4$-alkylthio groups and cyano, a $C_2$-$C_4$-alkenyl group, a $C_2$-$C_4$-alkinyl group, a pyridylmethyl group which can be substituted by halogen and/or methyl, a benzyl group which can be substituted by halogen and/or methyl, a formyl group, an alkylcarbonyl group having 1 or 2 carbon atoms in the alkyl structural unit which can be substituted by halogen, a phenylcarbonyl group which can be substituted by halogen and/or methyl, an alkoxy or alkylthiocarbonyl group having 1 to 4 carbon atoms in the alkyl structural unit, a phenoxycarbonyl group, a $C_1$-$C_4$-alkylsulphonyl group which can be substituted by halogen, or a phenylsulphonyl group which can be substituted by methyl, and

$R^3$ denotes a hydrogen atom, and

Z denotes a 5- or 6-membered heterocyclic group which contains two or three hetero atoms selected from amongst oxygen, sulphur and nitrogen atoms, at least one of these being a nitrogen atom, or denotes a 3-pyridyl group, the heterocyclic group and the 3-pyridyl group optionally being substituted by at least one substituent selected from amongst halogen atoms, alkyl groups having 1 to 4 carbon atoms, alkoxy groups having 1 to 4 carbon atoms, alkylthio groups having 1 to 4 carbon atoms, halogenoalkyl groups having 1 to 4 carbon atoms, halogenoalkoxy groups having 1 to 4 carbon atoms, alkylsulphonyl groups having 1 to 4 carbon atoms, the cyano group and the nitro group,

with the exception of the following compound:

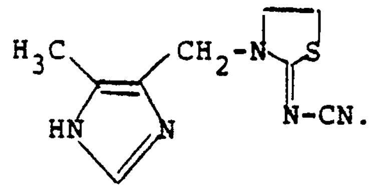

2. Compounds according to Claim 1, characterised in that

- R[1] denotes a hydrogen atom,
- A denotes an ethylene or trimethylene group,
- X denotes a sulphur atom or the group

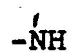

and

- Z denotes a 5- or 6-membered heterocyclic group which contains two hetero atoms selected from amongst oxygen, sulphur and nitrogen atoms, at least one of these being a nitrogen atom, or denotes a 3-pyridyl group, the heterocyclic group and the 3-pyridyl group optionally being substituted by at least one substituent selected from amongst the fluorine atom, the chlorine atom, the bromine atom, the methyl group, the methoxy group, the methylthio group, the trifluoromethyl group, the trifluoromethoxy group, the methylsulphonyl group, the cyano group and the nitro group, with the exception of the following compound:

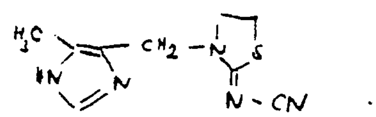

3. Heterocyclic compounds according to Claims 1 or 2, selected from amongst the following compounds:

1-(2-chloro-5-pyridylmethyl)-2-cyanoiminoimidazolidine of the formula

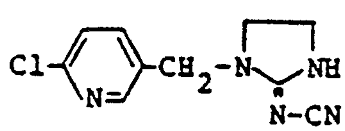

1-(2-fluoro-5-pyridylmethyl)-2-cyanoiminoimidazolidine of the formula

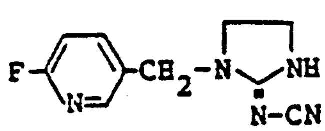

1-(2-chloro-5-pyridylmethyl)-2-cyanoiminotetrahydropyrimidine of the formula

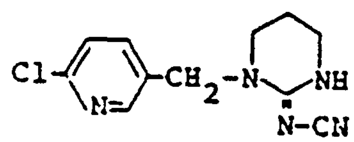

1-(2-methyl-5-pyridylmethyl)-2-cyanoiminoimidazolidine of the formula

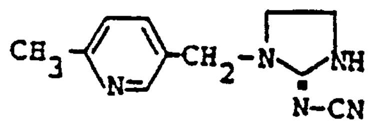

1-(2-chloro-5-thiazolylmethyl)-2-cyanoiminoimidazolidine of the formula

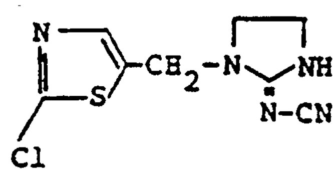

1-(2-chloro-5-thiazolylmethyl)-2-cyanoiminotetrahydropyrimidine of the formula

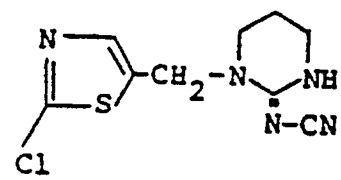

1-(2-methyl-5-pyrazinylmethyl)-2-cyanoiminoimidazolidine of the formula

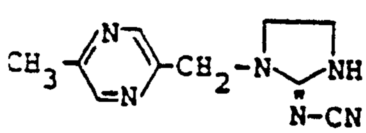

1-(2-chloro-5-pyridylmethyl)-2-cyanoiminothiazolidine of the formula

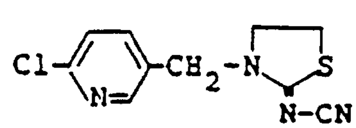

1-(2-chloro-5-pyridylmethyl)-2-cyanoiminotetrahydro-2H-1,3-thiazineof the formula

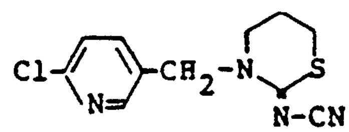

1-(2-chloro-5-thiazolylmethyl)-2-cyanoiminothiazolidine of the formula

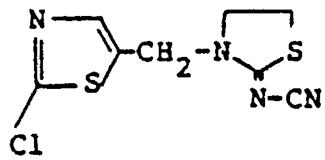

1-(2-methyl-5-pyrazinylmethyl)-2-cyanoiminothiazolidine of the formula

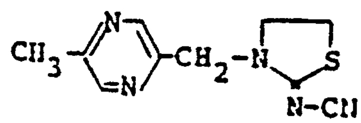

1-(2-methyl-5-thiazolylmethyl)-2-cyanoiminothiazolidine of the formula

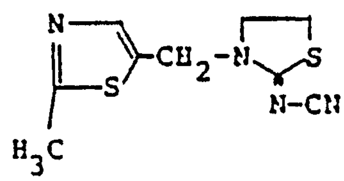

and 1-(1,2,5-thiadiazol-3-yl)-2-cyanoiminothiazolidine of the formula

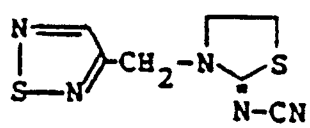

4. Insecticidal agents, characterised in that they contain at least one heterocyclic compound of the formula (I) according to Claim 1.

5. Method of controlling insect pests, characterised in that heterocyclic compounds of the formula (I) according to Claim 1 are allowed to act on the insect pests and/or their environment.

6. Use of novel heterocyclic compounds of the formula (I) according to Claim 1 for controlling insect pests.

7. Process for the preparation of insecticidal agents, characterised in that novel heterocyclic compounds of the formula (I) according to Claim 1 are mixed with extenders and/or surface-active agents.

## Revendications

1. Composés hétérocycliques répondant à la formule (I)

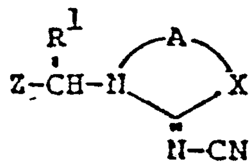

(I)

dans laquelle

$R^1$ représente un atome d'hydrogène ou un groupe méthyle;

A représente un groupe éthylène qui peut être substitué par un groupe méthyle ou bien, un groupe triméthylène qui peut être substitué par un groupe méthyle;

X représente un atome d'oxygène ou un atome de soufre ou encore le groupe

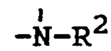

ou

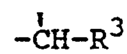

où

$R^2$ représente un atome d'hydrogène, un groupe alkyle en $C_1$-$C_4$ qui peut être substitué par un substituant choisi parmi des atomes d'halogène, des groupes alcoxy en $C_1$-$C_4$, des groupes alkyl(en $C_1$-$C_4$)thio et un groupe cyano, un groupe alcényle en $C_2$-$C_4$, un groupe alcynyle en $C_2$-$C_4$, un groupe pyridylméthyle qui peut être substitué par un atome d'halogène et/ou par un groupe méthyle, un groupe benzyle qui peut être substitué par un atome d'halogène et/ou par un groupe méthyle, un groupe formyle, un groupe alkylcarbonyle contenant 1 ou 2 atomes de carbone dans l'unité structurale alkyle, qui peut être substitué par un atome d'halogène, un groupe phénylcarbonyle qui peut être substitué par un atome d'halogène et/ou par un groupe méthyle, un groupe alcoxy- ou alkylthiocarbonyle contenant de 1 à 4 atomes de carbone dans l'unité structurale alkyle, un groupe phénoxycarbonyle, un groupe alkyl(en $C_1$-$C_4$)sulfonyle qui peut être substitué par un atome d'halogène ou un groupe phénylsulfonyle qui peut être substitué par un groupe méthyle; et

$R^3$ représente un atome d'hydrogène; et

Z représente un groupe hétérocyclique penta- ou hexagonal qui contient 2 ou 3 hétéro-atomes choisis parmi un atome d'oxygène, un atome de soufre et un atome d'azote, dont l'un au moins représente un atome d'azote, ou encore un groupe 3-pyridyle, le groupe hétérocyclique et le groupe 3-pyridyle étant éventuellement substitués par au moins un substituant choisi parmi le groupe comprenant des atomes d'halogène, des groupes alkyle contenant de 1 à 4 atomes de carbone, des groupes alcoxy contenant de 1 à 4 atomes de carbone, des groupes alkylthio contenant de 1 à 4 atomes de carbone, des groupes halogénalkyle contenant de 1 à 4 atomes de carbone, des groupes halogénalcoxy contenant de 1 à 4 atomes de carbone, des groupes alkylsulfonyle contenant de 1 à 4 atomes de carbone, le groupe cyano et le groupe nitro, à l'exception du composé ci-après

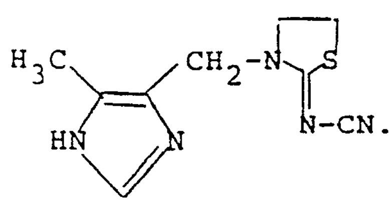

**2.** Composés selon la revendication 1, **caractérisés en ce que** :

$R^1$ représente un atome d'hydrogène,

A représente un groupe éthylène ou triméthylène,

X représente un atome de soufre ou le groupe

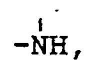

et

Z représente un groupe hétérocyclique penta- ou hexagonal qui contient 2 hétéro-atomes choisis parmi un atome d'oxygène, un atome de soufre et un atome d'azote, dont l'un au moins représente un atome d'azote, ou encore un groupe 3-pyridyle, le groupe hétérocyclique et le groupe 3-pyridyle étant éventuellement substitués par au moins un substituant choisi parmi un atome de fluor, un atome de chlore, un atome de brome, un groupe méthyle, un groupe méthoxy, un groupe méthylthio, un groupe trifluorométhyle, un groupe trifluorométhoxy, un groupe méthylsulfonyle, un groupe cyano et un groupe nitro,

à l'exception du composé ci-après

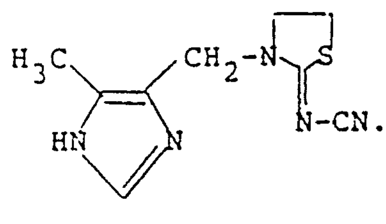

**3.** Composés hétérocycliques selon les revendications 1 et 2, choisis parmi les composés ci-après :

la 1-(2-chloro-5-pyridylméthyl)-2-cyanoiminoimidazolidine de formule

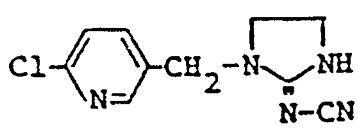

la 1-(2-fluoro-5-pyridylméthyl)-2-cyanoiminoimidazolidine de formule

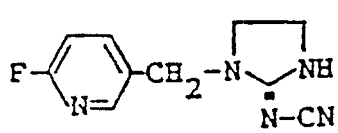

la 1-(2-chloro-5-pyridylméthyl)-2-cyanoiminotétrahydropyrimidine de formule

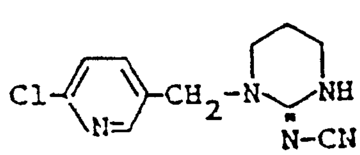

la 1-(2-méthyl-5-pyridylméthyl)-2-cyanoiminoimidazolidine de formule

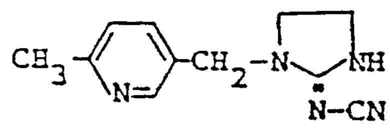

la 1-(2-chloro-5-thiazolylméthyl)-2-cyanoiminoimidazolidine de formule

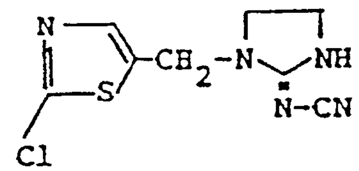

la 1-(2-chloro-5-thiazolylméthyl)-2-cyanoiminotétrahydropyrimidine de formule

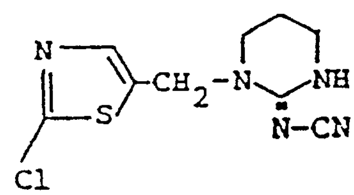

la 1-(2-méthyl-5-pyrazinylméthyl)-2-cyanoiminoimidazolidine de formule

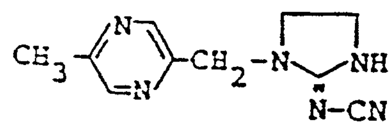

la 1-(2-chloro-5-pyridylméthyl)-2-cyanoiminothiazolidine de formule

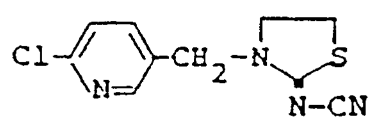

la 1-(2-chloro-5-pyridylméthyl)-2-cyanoiminotétra-hydro-2H-1,3-thiazine de formule

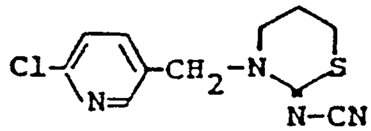

la 1-(2-chloro-5-thiazolylméthyl)-2-cyanoiminothiazolidine de formule

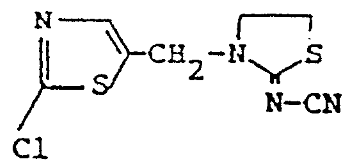

la 1-(2-méthyl-5-pyrazinylméthyl)-2-cyanoiminothiazolidine de formule

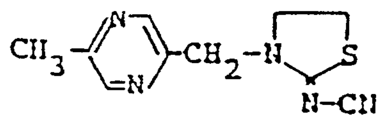

la 1-(2-méthyl-5-thiazolylméthyl)-2-cyanoiminothiazolidine de formule

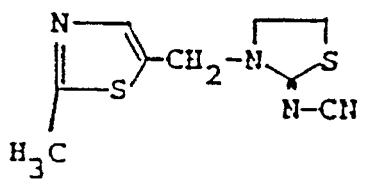

la 1-(1,2,5-thiadiazol-3-yl)-2-cyanoiminothiazolidine de formule

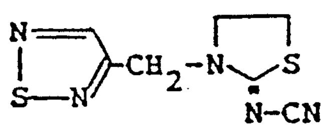

4. Agents insecticides **caractérisés en ce qu**'ils contiennent au moins un composé hétérocyclique de formule (I) selon la revendication 1.

5. Procédé pour lutter contre des insectes nuisibles, **caractérisé en ce qu**'on laisse agir des composés hétérocycliques de formule (I) selon la revendication 1, sur des insectes nuisibles et/ou leur biotope.

6. Utilisation de nouveaux composés hétérocycliques de formule (I) selon la revendication 1 pour lutter contre des insectes nuisibles.

7. Procédé pour l'utilisation d'agents insecticides, **caractérisé en ce qu**'on mélange de nouveaux composés hétérocycliques de formule (I) selon la revendication 1, avec des diluants et/ou des agents tensio-actifs.